# EUROPÄISCHE PATENTSCHRIFT

(11) **EP 2 910 204 B1**
(45) Veröffentlichungstag und Bekanntmachung des Hinweises auf die Patenterteilung: **04.05.2016**
(21) Anmeldenummer: 14156385.8
(22) Anmeldetag: 24.02.2014
(51) Int. Cl.: A61B 17/30

(54) **Medizinisches Instrument**
Medical instrument
Instrument médical

(43) Veröffentlichungstag der Anmeldung: 26.08.2015
(73) Patentinhaber: S & T AG, 8212 Neuhausen am Rheinfall (CH)
(72) Erfinder: Böhmdörfer, Richard, 79798 Jestetten (DE)
(74) Vertreter: Wagner, Kilian

(56) Entgegenhaltungen:
- WO-A1-2013/130365
- DE-A1-102007 047 059
- US-A1- 2012 137 472
- US-A1- 2013 175 067

## Beschreibung

Die vorliegende Erfindung betrifft ein medizinisches, insbesondere chirurgisches, Instrument mit einem Eingriffabschnitt für einen medizinischen Eingriff und einem zum Halten des Instrumentes ausgebildeten Griff mit einem Griffflächenabschnitt, der eine Anordnung mit Ausformungen und zwischen den Ausformungen gebildeten Einfassungsabschnitten aufweist.

Aus dem Stand der Technik sind zahlreiche medizinische Instrumente gemäß dem Oberbegriff des Patentanspruchs 1 bekannt. Entlang einer Längsachse des medizinischen Instrumentes sind an einem distalen (von dem dem Operateur abgewandten) Ende des medizinischen Instrumentes, auch als Instrumentenspitze bezeichnet, ein Eingriffabschnitt für einen medizinischen, insbesondere chirurgischen oder mikrochirurgischen, Eingriff und an einem proximalen Ende des medizinischen Instrumentes ein Griff angeordnet.

Diese Instrumente sind üblicherweise aufgrund von Hygienevorschriften vollständig aus einem rostfreien Metall, in der Regel Chromvanadium, Edelstahl oder Titan, hergestellt, das ohne eingebrachte Vertiefungen oder Erhebungen eine glatte Oberflächenstruktur aufweist.

Damit der Operateur das medizinische Instrument und insbesondere den Eingriffabschnitt für den medizinischen Eingriff gut handhaben kann, ohne dass die das medizinische Instrument betätigende Hand bzw. ein Finger des Operateurs von dem medizinischen Instrument abrutscht, sind die medizinischen Instrumente mit einem Griff mit einem Griffflächenabschnitt versehen.

In einer ersten Art von gattungsgemäß ausgebildeten medizinischen Instrumenten weist der Griffflächenabschnitt, als Ausformungen, in eine Oberfläche des Griffs gefräste längliche Rillen auf, zwischen denen das stehengebliebene, nicht weggefräste Material der Oberfläche des Griffes erhabene längliche Stege als Einfassungsabschnitte bildet. Dabei weisen die tiefen Rillen häufig jeweils eine Pyramidenform mit einem dreieckigen Querschnittsprofil auf, so dass zwischen den Querschnittsprofilen benachbarter Rillen ein rechter Winkel aufgespannt wird. Diese Rillen und Stege können ein Abrutschen der Hand und insbesondere des kraftaufbringenden Daumens des Operateurs besonders wirkungsvoll verhindern, wenn sie derart angeordnet sind, dass ihre jeweiligen Längsrichtungen mit der Längsachse des medizinischen Instrumentes einen Winkel von 60 Grad aufspannen. Dann sind die Rillen und Stege senkrecht zu einer Längsachse des auf dem Griff aufzubringenden Daumens ausgerichtet sind, dessen Längsachse bei gebräuchlichen medizinischen Instrumenten einen Winkel von 30 Grad mit der Längsachse des Instrumentes aufspannt.

In einer zweiten Art von gattungsgemäß ausgebildeten medizinischen Instrumenten weist der Griffflächenabschnitt, als Ausformungen, in der Oberfläche des Griffs eine erste Gruppe von zueinander parallelen länglichen Rillen und eine zweite Gruppe von zueinander parallelen länglichen Rillen auf, wobei die Rillen der ersten Gruppe mit den Rillen der zweiten Gruppe einen rechten Winkel aufspannen. Das stehengebliebene, nicht weggefräste Material der Oberfläche des Griffes, das von den Rillen der beiden Gruppen berandet wird, bildet eine Struktur von erhabenen rechteckförmigen Sockeln.

An medizinische und insbesondere chirurgische Instrumente werden besonders hohe Anforderungen hinsichtlich ihrer Reinigungseigenschaften und ihrer Griffigkeit gestellt. Bezüglich des Aspektes der Griffigkeit kommt erschwerend hinzu, dass der Operateur und Benutzer des medizinischen Instrumentes aus hygienischen Gründen für den eigenen Schutz und den des Patienten üblicherweise aus Latex gefertigte Handschuhe trägt. Der Griff des medizinischen Instrumentes muss daher so ausgebildet sein, dass der Griffflächenabschnitt im Kontakt mit dem die Hand des Operateurs umschließenden Handschuh das Aufbringen einer hohen Reibungskraft ohne ein Verrutschen des Handschuhs auf dem Griffflächenabschnitt und ohne ein Verrutschen eines in dem Handschuh aufgenommenen Fingers ermöglicht, der eine Kraft auf den Griffflächenabschnitt ausübt. Obwohl der Griffflächenabschnitt zum Aufbringen einer hohen Haftreibung und im Falle eines beginnenden Verrutschens zum Aufbringen einer hohen Gleitreibung ausgebildet sein muss, muss der Griffflächenabschnitt so ausgestaltet sein, dass er den Handschuh des Operateurs nicht beispielsweise durch scharfkantige Übergänge zwischen den Stegen und Rillen beschädigt. Außerdem muss der Griffflächenabschnitt so ausgebildet sein, dass der Handschuh nicht am Griffflächenabschnitt haftet oder sich in den Rillen des Griffflächenabschnitts verkeilt, wenn sich die Finger des Operateurs von dem medizinischen Instrument lösen.

Hinsichtlich der Reinigungseigenschaften wird bei medizinischen Instrumenten vor allem Wert darauf gelegt, dass an dem Instrument, insbesondere an dem zur Erzielung einer guten Griffigkeit mit Rillen oder anderen Vertiefungen versehenen und somit hinsichtlich der Reinigungseigenschaften besonders kritischen Griffflächenabschnitt, anhaftende Verunreinigungen, wie beispielsweise angetrocknetes Blut, mühelos entfernt werden können. Da medizinische Instrumente nach einer groben Reinigung anschließend in einem Autoklaven dampfsterilisiert werden, sollte das medizinische Instrument so ausgestaltet sein, dass nach einer erfolgten Dampfsterilisation sich in oder auf dem Instrument keine Restflüssigkeitsmengen sammeln.

Die im Stand der Technik bekannten gattungsgemäßen medizinischen Instrumente stellen einen Kompromiss zwischen dem Erreichen einer guten Griffigkeit des Instrumentes und guter Reinigungseigenschaften des Instrumentes dar.

Bei der oben erwähnten ersten Art von gattungsgemäß ausgebildeten medizinischen Instrumenten leidet die Griffigkeit allerdings dann, wenn die Kraft entlang der Oberfläche des Griffflächenabschnitts nicht in senkrechter Richtung zu den parallel zueinander angeordneten Rillen und Stegen aufgebracht wird. In diesem Fall ist ein sicheres Führen des medizinischen Instrumentes durch den Operateur gefährdet, da die auf den Rillen und Stegen aufgebrachten Finger des Operateurs in Richtung der jeweiligen Längsrichtungen der Rillen und Stege verrutschen können. Im Falle eines derartigen Verrutschens droht außerdem die Gefahr, dass die Stege eine messerklingenartige Wirkung entfalten, und der Handschuh durch die durch das Verrutschen bewirkte Bewegung entlang der Längsrichtung der Stege beschädigt wird. Zudem sind die tief ausgeprägten, im rechten Winkel aufeinander treffenden Rillen schwer zu reinigen.

Bei der oben erwähnten zweiten Art von gattungsgemäß ausgebildeten medizinischen Instrumenten leiden die Reinigungseigenschaften vor allem darunter, dass die rechteckförmig ausgebildeten Sockel vollständig von Rillen umgeben sind, die ein maschenartiges Muster von sich rechtwinklig kreuzenden und somit schlecht zu reinigenden Rillenabschnitten ausbilden. Zudem droht bei einer zu engen Beabstandung der erhabenen Sockel voneinander ein Verklemmen des Handschuhs zwischen den Sockeln.

Ferner kann es bei beiden Arten von gattungsgemäß ausgebildeten medizinischen Instrumenten nach einer Dampfsterilisation zu Flüssigkeitsansammlungen in oder auf den Rillen des Instrumentes kommen.

Die DE 10 2007 047 059 A1 beschreibt ein medizinisches Instrument gemäß dem Oberbegriff des Anspruchs 1, mit einem Griffflächenabschnitt, der mit bevorzugt langgestreckten Vertiefungen versehen sein kann. Die Griffigkeit scheint verbesserungswürdig.

Die US 2012/0137472 A1, die US 2013/0175067 A1 und die WO 2013/130365 A1 beschreibt einen fachfremden Stand der Technik, in welchem unterschiedliche Griffkonzepte für Nicht-Medizingeräte erläutert sind.

Die Aufgabe der vorliegenden Erfindung besteht daher in der Bereitstellung eines medizinischen Instrumentes mit einer optimierten Griffigkeit, so dass das medizinische Instrument vom Operateur sicher und ohne die Gefahr eines Verrutschens einer das Instrument haltenden Hand auf dem Instrument geführt werden kann, einer guten Lösbarkeit des Handschuhs von dem Griffflächenabschnitt und optimierten Reinigungseigenschaften im Vergleich zum Stand der Technik.

Die Aufgabe der vorliegenden Erfindung wird durch ein gattungsgemäßes medizinisches Instrument mit den kennzeichnenden Merkmalen des unabhängigen Patentanspruchs 1 gelöst. Zusätzliche Merkmale weiterer vorteilhafter Ausführungsformen ergeben sich aus den abhängigen Patentansprüchen.

Das erfindungsgemäße medizinische, insbesondere chirurgische oder mikrochirurgische, Instrument ist mit einem Eingriffabschnitt für einen medizinischen Eingriff und einem zum Halten des Instrumentes, bevorzugt zur Betätigung des Eingriffabschnitts, ausgebildeten Griff mit einem Griffflächenabschnitt versehen, der eine Anordnung mit Ausformungen und zwischen den Ausformungen gebildeten Einfassungsabschnitten aufweist. Der Eingriffabschnitt ist nicht-beweglich oder beweglich, beispielsweise als Pinzette oder Zange, oder elektrisch aktivierbar ausgebildet. Erfindungsgemäß weisen die Ausformungen jeweils einen hexagonalförmigen Rand mit sechs Randabschnitten gleicher Länge auf. Dabei spannen aneinander angrenzende Randabschnitten der Randabschnitte jeder Ausformung vor und/oder nach einer Abwicklung der Anordnung auf eine ebene Fläche denselben Winkel von 120 Grad auf. Der hexagonalförmige Rand ist durch die zwischen benachbarten der Ausformungen gebildete, bevorzugt rechteckig konstruierte, Einfassungsabschnitte ausgebildet, wodurch sich auf dem Griffflächenabschnitt eine Wabenstruktur ergibt. Bei der bevorzugten Ausbildung mit den rechteckig konstruierten Einfassungsabschnitten begrenzen drei winklig zueinander angeordnete rechteckige Einfassungsabschnitte einen dreieckförmigen Abschnitt.

In einer Ausführungsform sind die Ausformungen des Griffflächenabschnitts als Vertiefungen, oder mit anderen Worten als Ausnehmungen, Eindellungen, Einprägungen oder als Einwölbungen, in dem Griffflächenabschnitt ausgebildet. Die Einfassungsabschnitte des Griffflächenabschnitts sind gegenüber den als die Vertiefungen ausgebildeten Ausformungen des Griffflächenabschnitts erhaben. In dieser Ausführungsform greifen die Fingerkuppen bzw. die diese umgebenden Teile des Handschuhs des das medizinische Instrument haltenden Operateurs in die Vertiefungen mit dem jeweils hexagonalförmig ausgebildeten Rand, begleitet von einer entsprechenden elastischen Verformung der Fingerkuppen, und liegen auf den erhaben ausgebildeten Einfassungsabschnitten auf.

Dadurch dass jede als Vertiefung ausgebildete Ausformung sechs in erhabene Einfassungsabschnitte übergehende Randabschnitte aufweist, die auf einer von drei zueinander um 60 Grad (bzw. 120 Grad) versetzten Achsen angeordnet sind, ermöglicht der erfindungsgemäße Griffflächenabschnitt dieser Ausführungsform eine sehr hohe Griffigkeit des Instrumentes ohne besondere Beachtung einer Richtung einer Kraftaufbringung durch den das medizinische Instrument haltenden Operateur. Neben der sehr hohen Griffigkeit und damit der reduzierten Verrutschgefahr bewirken die Vertiefungen aufgrund ihres hexagonalförmigen Randes auch, dass die auf den Griffflächenabschnitt kontaktierende Flächen des Handschuhs des Operateurs ausgeübte Kraft gleichmäßig auf viele Kontaktstellen verteilt wird, so dass die Gefahr einer Beschädigung des Handschuhs im Vergleich zu den bekannten, mit Rillen versehenen Griffflächenabschnitten reduziert wird.

Da benachbarte Randabschnitte einer Vertiefung mit hexagonalförmigem Rand einen stumpfen und somit weiten Winkel von 120 Grad aufspannen, weisen die erfindungsgemäß ausgebildeten Vertiefungen gute Reinigungseigenschaften auf. Darüber hinaus ermöglicht die erfindungsgemäße Anordnung der Vertiefungen, einer Ansammlung einer Restflüssigkeit nach erfolgter Dampfsterilisation des Instrumentes vorzubeugen.

In einer weiteren Ausführungsform sind die Ausformungen des Griffflächenabschnitts als Auswölbungen, oder mit anderen Worten als Ausbeulungen, Erhebungen oder Hügel, auf dem Griffflächenabschnitt ausgebildet. Die Einfassungsabschnitte des Griffflächenabschnitts sind gegenüber den als die Auswölbungen ausgebildeten Ausformungen des Griffflächenabschnitts vertieft ausgebildet. In dieser Ausführungsform greifen die Fingerkuppen bzw. die diese umgebenden Flächen des Handschuhs des das medizinische Instrument haltenden Operateurs in die Einfassungsabschnitte, die den hexagonalförmigen Rand der als Auswölbungen ausgebildeten Ausformungen bilden, begleitet von einer entsprechenden elastischen Verformung der Fingerkuppen, und liegen auf den Auswölbungen auf, ebenfalls begleitet von einer entsprechenden elastischen Verformung der Fingerkuppen.

Dadurch dass jede als Auswölbung ausgebildete Ausformung sechs in vertiefte Einfassungsabschnitte übergehende Randabschnitte aufweist, die auf einer von drei zueinander um 60 Grad (bzw. 120 Grad) versetzten Achsen angeordnet sind, ermöglicht der erfindungsgemäße Griffflächenabschnitt dieser Ausführungsform eine sehr hohe Griffigkeit des Instrumentes ohne besondere Beachtung einer Richtung einer Kraftaufbringung durch den das medizinische Instrument haltenden Operateur. Neben der sehr hohen Griffigkeit und damit der reduzierten Verrutschgefahr bewirken die Auswölbungen aufgrund ihres hexagonalförmigen Randes auch, dass die auf den Griffflächenabschnitt kontaktierende Flächen des Handschuhs des Operateurs ausgeübte Kraft gleichmäßig auf viele Kontaktstellen, beim Griffflächenabschnitt gebildet durch die Auswölbungen und deren in die vertieften Einfassungsabschnitte übergehenden Randabschnitte, verteilt wird, so dass die Gefahr einer Beschädigung des Handschuhs vermindert wird.

Da die die Auswölbungen umgebenden Einfassungsabschnitte mit ihrem jeweils benachbarten Einfassungsabschnitt einen stumpfen und somit weiten Winkel von 120 Grad aufspannen, bietet diese Ausführungsform gute Reinigungseigenschaften und kann eine Ansammlung einer Restflüssigkeit nach erfolgter Dampfsterilisation des Instrumentes vermeiden.

Falls die Anordnung auf einem ebenen Griffflächenflächenabschnitt, der ohne Betrachtung der Ausformungen eine ebene Oberfläche aufweist, angeordnet ist, so spannen die aneinander angrenzenden Randabschnitten der Randabschnitte jeder Ausformung vor und nach der Abwicklung der Anordnung auf eine ebene Fläche denselben Winkel von 120 Grad auf. Für einen ebenen Griffflächenabschnitt ist folglich keine Fallunterscheidung bezüglich der Abwicklung erforderlich und es ergibt sich ein und dieselbe Ausführungsform.

Falls die Anordnung auf einem gekrümmten Griffflächenflächenabschnitt angeordnet ist, also auf einem Griffflächenabschnitt, der ohne Betrachtung der Ausformungen eine gekrümmte bzw. gebogene Oberfläche aufweist, so sind verschiedene Ausführungsformen möglich.

In einer Ausführungsform ist spannen die aneinander angrenzenden Randabschnitte der Randabschnitte jeder Ausformung vor der Abwicklung von der gekrümmten Oberfläche des Griffflächenabschnitts auf eine ebene Fläche denselben Winkel von 120 Grad auf. Bei dieser Ausführungsform sind die Ausformungen folglich so im Griffflächenabschnitt angeordnet, dass - bei Betrachtung einer einzelnen Ausformung - die sechs Randabschnitte gleicher Länge, die den hexagonalförmigen Rand der Ausformung bilden, sich auf dem gekrümmten oder gebogenen Griffflächenabschnitt des Griffs in einer für die betrachtete Ausformung spezifischen, ebenen Fläche erstrecken, so dass die Randabschnitte streng geradlinig verlaufen und benachbarte Randabschnitte der Ausformungen denselben Winkel von 120 Grad aufspannen, und so dass die Randabschnitte nicht entsprechend einer Krümmung der gekrümmten Oberfläche des Griffflächenabschnitts gebogen sind und/oder die benachbarten Randabschnitte der Ausformung nicht über ihre gesamte Länge hinweg in derselben Ebene verlaufen. Dies gilt entsprechend für die anderen der Ausformungen, deren jeweilige sechs Randabschnitte gleicher Länge sich auf dem gekrümmten oder gebogenen Griffflächenabschnitt des Griffs in einer für die betrachtete Ausformung spezifischen, ebenen Fläche erstrecken, die sich von der spezifischen ebenen Fläche einer anderen Ausformung unterscheiden kann.

Diese Ausführungsform ermöglicht auch dann zueinander identische Ausbildungen der Ausformungen und damit näherungsweise über den gesamten Griffflächenabschnitt konstant ausgeprägte Eigenschaften hinsichtlich der Griffigkeit und Reinigung, wenn der Griffflächenabschnitt keinen konstanten Krümmungsradius aufweist.

In einer anderen Ausführungsform spannen die aneinander angrenzenden Randabschnitte der Randabschnitte jeder Ausformung nach der Abwicklung von der gekrümmten Oberfläche des Griffflächenabschnitts auf eine ebene Fläche denselben Winkel von 120 Grad auf. Bei dieser Ausführungsform sind die Ausformungen folglich so im Griffflächenabschnitt angeordnet, dass - bei Betrachtung einer einzelnen Ausformung - die sechs Randabschnitte gleicher Länge, die den hexagonalförmigen Rand der Ausformung bilden, sich auf dem gekrümmten oder gebogenen Griffflächenabschnitt des Griffs in einer für die betrachtete Ausformung spezifischen Fläche erstrecken, die ein Ausschnitt der gekrümmten oder gebogenen Oberfläche des Griffflächenabschnitts darstellt, so dass die Randabschnitte entsprechend einer Krümmung der gebogenen Oberfläche des Griffflächenabschnitts gebogen sind und/oder die benachbarten Randabschnitte der Ausformung nicht über ihre gesamte Länge hinweg in derselben Ebene verlaufen. Dies gilt entsprechend für die anderen der Ausformungen.

Diese Ausführungsform ermöglicht aufgrund der Randabschnitte, die einer Krümmung der gebogenen Oberfläche des Griffflächenabschnitts entsprechend gebogen ausgestaltet sind, für jede Ausformung den Übergang von der Ausformung zu den sie umgebenden Einfassungsabschnitten an die jeweilige Krümmung der gebogenen Oberfläche des Griffflächenabschnitts anzupassen. Dadurch können bei starken Krümmungen scharfkantige Übergange von der Ausformung zu den sie umgebenden Einfassungsabschnitten vermieden werden.

In einer weiteren erfindungsgemäßen Ausführungsform sind die Ausformungen so angeordnet, dass, vorzugsweise für alle der Ausformungen, ein Paar der zueinander parallelen der Randabschnitte einer der Ausformungen mit einer für die Verwendung des Instrumentes vorgesehenen Halteachse, insbesondere eine Längsachse des Griffes bzw. Griffflächenabschnitts und/oder des Instrumentes, einen rechten Winkel aufspannt. Mit der Halteachse ist beispielsweise die Achse gemeint, entlang derer bei einer bestimmungsgemäßen Verwendung des medizinischen Instrumentes der Daumen des Operateurs auf dem Griffflächenabschnitt aufgelegt wird.

Durch diese senkrechte Anordnung, in einer Ebene des Griffflächenabschnitts, eines Paares der insgesamt drei Paare von parallelen Randabschnitten jeder der Ausformungen zu der Halteachse kann die von dem Griffflächenabschnitt bereitgestellte Griffigkeit für eine in Richtung der Halteachse aufgebrachte Kraft optimiert werden.

In noch einer weiteren erfindungsgemäßen Ausführungsform sind die Ausformungen so angeordnet, dass eine Winkelhalbierende der aneinander angrenzenden der Randabschnitte der Ausformungen sich parallel zu einer Längsachse des Griffes bzw. Griffflächenabschnitts und/oder des Instrumentes erstreckt.

Dann spannt ein Paar der insgesamt drei Paare von parallelen Randabschnitten jeder der Ausformungen einen rechten Winkel mit der Längsachse des auf dem Griff aufzubringenden Daumens auf, die bei gebräuchlichen medizinischen Instrumenten einen Winkel von 30 Grad mit der Längsachse des Griffes bzw. Griffflächenabschnitts und/oder Instrumentes aufspannt. Die von dem Griffflächenabschnitt bereitgestellte Griffigkeit kann somit für eine in Richtung einer für den Daumen üblichen Halteachse aufgebrachte Kraft optimiert werden. Als weiterer Vorteil ergibt sich, dass das medizinische Instrument auch bei einer Drehung um 180 Grad um seine Längsachse eine unverändert gute Griffigkeit aufweist, da auch dann ein Paar von parallelen Randabschnitten jeder der Ausformungen einen rechten Winkel mit der Längsachse des auf dem Griff aufzubringenden Daumens aufspannt. Dies stellt eine deutliche Verbesserung gegenüber der oben erwähnten ersten Art von gattungsgemäß ausgebildeten medizinischen Instrumenten aus dem Stand der Technik dar, die bei einer Drehung um 180 Grad um ihre Längsachse an Griffigkeit einbüßen, da dann die Rillen nicht mehr einen rechten Winkel mit der üblichen Halteachse von 30 Grad zur Längsachse des Instrumentes aufspannen.

In noch einer weiteren erfindungsgemäßen Ausführungsform verläuft, vorzugsweise für alle der Ausformungen, einer der Randabschnitte einer der Ausformungen zu einem benachbarten der Randabschnitte einer anderen der Ausformungen parallel und weist ein zwischen diesen benachbarten parallelen Randabschnitten angeordneter Einfassungsabschnitt der Einfassungsabschnitte eine konstante, senkrecht zu den parallelen Randabschnitten gemessene Einfassungsabschnittbreite auf.

Dadurch ergibt sich eine besonders regelmäßige Wabenstruktur mit gleichmäßig hoher Griffigkeit und gleich guten Reinigungseigenschaften auf dem gesamten Griffflächenabschnitt.

Eine die zuletzt aufgeführte erfindungsgemäße Ausführungsform weiterentwickelnde und verbessernde Ausführungsform ist derart ausgestaltet, dass zusätzlich zu den zuvor erwähnten Merkmalen der zuletzt aufgeführten erfindungsgemäßen Ausführungsform alle zwischen benachbarten parallelen Randabschnitten der Randabschnitte angeordneten Einfassungsabschnitte der Einfassungsabschnitte eine konstante, vorzugsweise identische, Einfassungsabschnittbreite im Bereich von 0,4 mm bis 3,7 mm, bevorzugt von 0,5 bis 3,5, noch bevorzugter von 0,6 mm bis 1,6 mm, aufweisen, und/oder jede der Ausformungen zwischen ihren jeweils gegenüberliegenden parallelen Randabschnitten eine konstante, vorzugsweise zu den anderen Ausformungen identische, Breite im Bereich von 0,7 mm bis 5,7 mm, bevorzugt 0,8 mm bis 5,5 mm, noch bevorzugter von 1,0 mm bis 2,4 mm, aufweist.

Die sich dadurch ergebende, besonders regelmäßige Wabenstruktur mit den oben aufgeführten Abmessungen bietet eine für medizinische Instrumente optimierte Griffigkeit und für medizinische Instrumente optimierte Reinigungseigenschaften auf dem gesamten Griffflächenabschnitt.

Eine die zuletzt aufgeführte erfindungsgemäße Ausführungsform nochmals weiterentwickelnde und verbessernde Ausführungsform ist derart ausgestaltet, dass zusätzlich zu den zuvor erwähnten Merkmalen der zuletzt aufgeführten erfindungsgemäßen Ausführungsform das Verhältnis zwischen der Breite der Ausformungen und der Einfassungsabschnittbreite im Bereich von 1,4 bis 1,8 liegt und bevorzugt dem Goldenen Schnitt entspricht. Mit einem dem Goldenen Schnitt entsprechenden Verhältnis ist dabei das Verhältnis der Goldenen Zahl zu der Zahl eins gemeint, also näherungsweise 1,618 : 1. Die Goldene Zahl kann als irrationale Zahl nur näherungsweise angegeben werden. Daher gelten im Rahmen der vorliegenden Erfindung Zahlen, die weniger als 5 Prozent von der mathematischen Definition der Goldenen Zahl abweichen, als die Goldene Zahl. Die Goldene Zahl ergibt sich auch aus dem Verhältnis eines sehr hohen Gliedes der Fibunacci-Folge zu dem vorherigen Glied dieser Folge.

Die sich dadurch ergebende, besonders regelmäßige Wabenstruktur mit den oben aufgeführten Abmessungen und Verhältnissen bietet eine für medizinische Instrumente nochmals optimierte Griffigkeit und für medizinische Instrumente nochmals optimierte Reinigungseigenschaften auf dem gesamten Griffflächenabschnitt.

Eine weitere erfindungsgemäße Ausführungsform ist derart ausgestaltet, dass die Ausformungen, insbesondere untereinander einheitlich, als Vertiefung, mit anderen Worten als Ausnehmungen, Eindellungen, Einprägungen oder als Einwölbungen ausgedrückt, oder als Auswölbung, mit anderen Worten als Ausbeulungen, Erhebungen oder Hügel ausgedrückt, gegenüber einer Ebene, auf der der hexagonalförmige Rand der jeweiligen der Ausformungen liegt, ausbildet sind und sich, insbesondere untereinander einheitlich, maximal im Bereich von 0,2 mm bis 1,4 mm, bevorzugt von 0,35 mm bis 1,20 mm, senkrecht zu der Ebene, auf der der hexagonalförmige Rand der jeweiligen der Ausformungen liegt, erstrecken.

Diese Ausbildung der Ausformungen ermöglicht eine sehr gute Griffigkeit und gute Reinigungseigenschaften.

Ergänzend zu der zuletzt aufgeführten erfindungsgemäßen Ausführungsform ist optional oder alternativ zumindest eine Untermenge der Ausformungen als Durchgangslöcher ausgebildet.

Dies steigert vor allem die Reinigungseigenschaften des medizinischen Instrumentes und beugt einer Ansammlung von Restflüssigkeiten auf dem Instrument nach einer Dampfsterilisation vor.

Eine weitere erfindungsgemäße Ausführungsform ist derart ausgestaltet, dass die Ausformungen, insbesondere untereinander einheitlich, gewölbt sind. Dabei sind die Auswölbungen bevorzugt entweder als konkave Vertiefung oder als konvexe Auswölbung ausgebildet. Der Krümmungsradius bestimmt dabei das Ausmaß der Vermeidung einer Ansammlung von Restflüssigkeit nach einer Dampfsterilisation.

Diese gewölbte Ausbildung ermöglicht eine Steigerung der Reinigungseigenschaften und nach einer Dampfsterilisation ein Unterbinden der Ansammlung von Restflüssigkeit auf dem medizinischen Instrument. Außerdem wird durch die konkaven Vertiefungen oder die konvexen Auswölbungen auf das medizinische Instrument auftreffendes Licht gestreut und abgelenkt, so dass im Vergleich zu den bekannten gattungsgemäßen Instrumenten die durch ein auf das Instrument einfallendes Licht bewirkte Blendwirkung reduziert werden kann.

Eine weitere erfindungsgemäße Ausführungsform ist derart ausgestaltet, dass, insbesondere einheitlich für alle der Einfassungsabschnitte und alle der Ausformungen, ein Übergangsbereich von einem der Einfassungsabschnitte zu einer der Ausformungen kantig oder abgerundet ausgebildet ist.

Ein kantig ausgebildeter Übergangsbereich ermöglicht eine Steigerung der Griffigkeit, während ein abgerundet ausgebildeter Übergangsbereich die Reinigungseigenschaften und die Vermeidung einer Ansammlung von Restflüssigkeit nach einer Dampfsterilisation fördert.

Alternativ oder ergänzend zu der zuletzt aufgeführten erfindungsgemäßen Ausführungsform spannt, insbesondere einheitlich für alle der Einfassungsabschnitte und alle der Ausformungen, ein Wandabschnitt einer der Einfassungsabschnitte mit einem dazu durch den Übergangsbereich beabstandeten Wandabschnitt einer der Ausformungen einen rechten oder stumpfen Winkel auf.

Ein aufgespannter rechter Winkel ermöglicht eine Steigerung der Griffigkeit, während ein stumpfer Winkel die Reinigungseigenschaften und die Vermeidung einer Ansammlung von Restflüssigkeit nach einer Dampfsterilisation fördert.

Gemäß einer weiteren vorteilhaften erfindungsgemäßen Ausführungsform ist der Griffflächenabschnitt in mindestens zwei voneinander in einem Winkel von mindestens 20 Grad in einer zu einer Längsachse des Instrumentes radialen Umfangsrichtung beabstandete Griffflächenteilabschnitte unterteilt, und jeder Griffflächenteilabschnitt ist in der radialen Umfangsrichtung auf mindestens 10 Prozent der gesamten radialen Umfangsrichtung angeordnet.

Durch die Unterteilung des Griffflächenabschnitts kann die Griffigkeit im Vergleich zu einem Griffflächenabschnitt ohne Unterteilung gesteigert werden, da die Griffflächenteilabschnitte gezielt für unterschiedliche Finger des Operateurs auf dem Griff angeordnet werden können. Aufgrund der gezielteren und somit effizienteren Bereitstellung der Griffflächenteilabschnitte anstelle eines einzelnen großflächigen Griffflächenabschnitts können die Reinigungseigenschaften verbessert werden.

Eine weitere erfindungsgemäße Ausführungsform ist derart ausgestaltet, dass der Griffflächenabschnitt in benachbarte Griffflächenteilabschnitte unterteilt ist, und einer der Griffflächenteilabschnitte ausschließlich als Vertiefungen ausgebildete Ausformungen aufweist, während der andere der Griffflächenteilabschnitte ausschließlich als Auswölbungen ausgebildete Ausformungen aufweist.

Dies ermöglicht eine kombinierte Nutzung der beiden grundsätzlich möglichen Ausgestaltungsformen der Ausformungen, so dass ein derartig ausgebildeter Griffflächenabschnitt die Gesamtheit der individuellen Vorteile beider Ausformungsarten bieten kann.

Weitere vorteilhafte erfindungsgemäße Ausführungsformen ergeben sich aus der Kombination von mindestens zwei der zuvor oder im nachfolgenden aufgeführten erfindungsgemäßen Ausführungsformen, sofern eine derartige Kombination nicht offensichtlich widersinnig ist.

Hinsichtlich der im Rahmen dieser Offenbarung genannten Winkelmaße und Angaben gleicher Längen gilt eine Abweichung von bis zu 2 Grad für die Winkelmaße und von bis zu 2 Prozent bezüglich der Längen als Einhaltung der genannten Maße und Angaben. Das Winkelmaß des vollen Kreises, auch Vollwinkel genannt, beträgt 360 Grad.

Die erfindungsgemäßen Instrumente einschließlich des Griffflächenabschnitts sind aus einem rostfreien Metall, beispielsweise Chromvanadium, Edelstahl oder Titan, hergestellt. Teile dieser Instrumente können aber auch aus Kunststoffen oder anderen Materialien gefertigt sein.

In einer bevorzugten Ausführungsform ist das erfindungsgemäße medizinische Instrument eine neurochirurgische Pinzette, die mit dem Griffflächenabschnitt mit der erfindungsgemäßen Hexagonalstruktur versehen ist. Dadurch kann auch bei diesem besonders filigranen Instrument eine gute Haftung der Hand des Operateurs auf dem Griffflächenabschnitt gewährleistet werden.

Im Folgenden werden beispielhafte Ausführungsformen der Erfindung anhand der beigefügten Figuren erläutert, in denen
- Fig. 1: eine Draufsicht einer erfindungsgemäßen Anordnung mit Ausformungen und zwischen den Ausformungen ausgebildeten Einfassungsabschnitten zeigt;
- Fig. 2A: eine Querschnittansicht entlang einer in der Fig. 1 dargestellten Schnittlinie C-C' für eine Ausführungsform zeigt, bei der die Ausformungen einheitlich als konkave Vertiefungen ausgebildet sind;
- Fig. 2B: eine Querschnittansicht entlang der in der Fig. 1 dargestellten Schnittlinie C-C' für eine andere Ausführungsform zeigt, bei der die Ausformungen einheitlich als konvexe Auswölbungen ausgebildet sind;
- Fig. 3: unterschiedliche Ausführungsformen hinsichtlich des Übergangsbereichs von einem der Einfassungsabschnitte zu einer der Ausformungen und hinsichtlich des zwischen einem Wandabschnitt einer der Einfassungsabschnitte und einem mit einem dazu durch den Übergangsbereich beabstandeten Wandabschnitt einer der Ausformungen aufgespannten Winkel zeigt;
- Fig. 4: einen Griff eines medizinischen Instrumentes mit zwei Griffflächenteilabschnitten zeigt;
- Fig. 5A: eine perspektivische Ansicht eines beispielhaften erfindungsgemäßen Instrumentes zeigt; und
- Fig. 5B: eine Seitenansicht des beispielhaften erfindungsgemäßen Instrumentes von Fig. 5A zeigt.

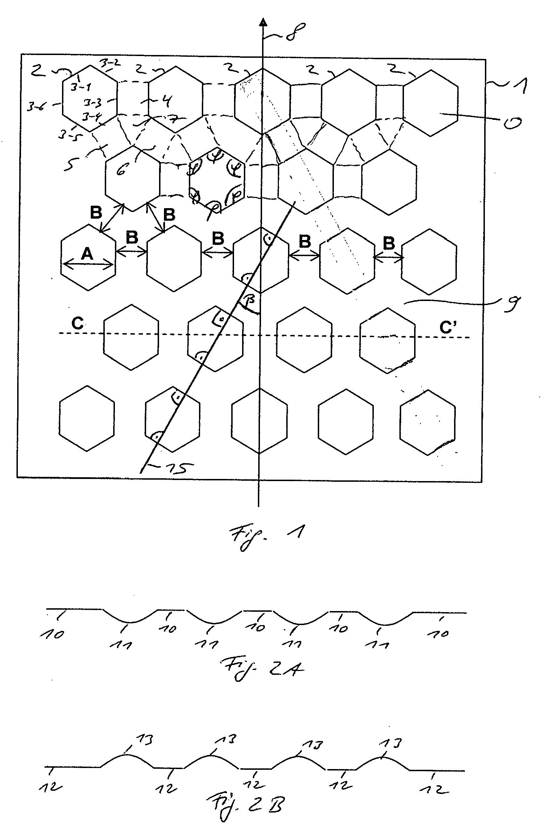

Fig. 1 zeigt eine Draufsicht einer erfindungsgemäßen Anordnung mit Ausformungen und zwischen den Ausformungen ausgebildeten Einfassungsabschnitten. Der Griffflächenabschnitt 1 umfasst mehrere Ausformungen 0 mit jeweils einem hexagonalförmigen Rand 2 mit sechs Randabschnitten 3-1, 3-2, 3-3, 3-4, 3-5 und 3-6 gleicher Länge, wobei aneinander angrenzende dieser Randabschnitte einer der Ausformungen 0 denselben Winkel ϕ = 120 Grad aufspannen. Zwischen benachbarten, zueinander parallel verlaufenden Randabschnitten benachbarter Ausformungen sind rechteckige Einfassungsabschnitte 4, 5 und 6 mit zueinander unterschiedlich ausgerichteten Seiten ausgebildet. Je eine Seite dieser drei rechteckigen Einfassungsabschnitte 4, 5 und 6 begrenzt einen dreieckigen Einfassungsabschnitt 7, so dass die rechteckigen Einfassungsabschnitte 4, 5 und 6 mit zueinander unterschiedlich ausgerichteten Seiten den dreieckigen Einfassungsabschnitt 7 umschließen. Die Ausformungen 0 haben eine zueinander einheitliche Breite A, die jeweils durch den Abstand der gegenüberliegenden Randabschnitte, beispielsweise 3-1 und 3-4 bzw. 3-2 und 3-5 bzw. 3-3 und 3-6, einer jeweiligen Ausformung 0 definiert ist. Benachbarte parallele Randabschnitte benachbarter Ausformungen sind einheitlich mit der Einfassungsabschnittbreite B voneinander beabstandet. Der in Fig. 1 gezeigte Griffflächenabschnitt weist eine regelmäßige Wabenstruktur 9 auf. Ferner ist eine Winkelhalbierende der aneinander grenzenden Randabschnitte, beispielsweise 3-1 und 3-2 bzw. 3-4 und 3-5, einer Ausformung 0 parallel zu einer Längsachse des Instrumentes 8, deren in Fig. 1 gezeichnete Pfeilspitze in Richtung der Instrumentenspitze zeigt. Ferner ist in Fig. 1 die zur Verwendung des Instrumentes vorgesehene Halteachse 15 unter der Annahme des zuvor erwähnten, häufig anzutreffenden Haltewinkels β = 30 Grad eingezeichnet. Die Randabschnitte 3-1 und 3-5 jeder der Ausformungen 0 verlaufen senkrecht zu der Halteachse 15.

Der in Fig. 1 gezeigte Griffflächenabschnitt 1 umfasst nur Ausformungen mit einem vollständig geschlossenen und hexagonalförmigen Rand. In einer anderen Ausführungsform kann der Griffflächenabschnitt an seinen Rändern durch Ausformungen begrenzt sein, die keinen vollständig geschlossenen hexagonalförmigen Rand sondern nur einen geöffneten Rand mit weniger als sechs Randabschnitten, beispielsweise mit drei Randabschnitten, haben.

Fig. 2A zeigt eine Querschnittansicht entlang einer in der Fig. 1 dargestellten Schnittlinie C-C' für eine Ausführungsform, bei der die Ausformungen einheitlich als konkave Vertiefungen ausgebildet sind. Die Ausformungen 0 sind als konkave Vertiefungen 11 ausgebildet, und die Einfassungsabschnitte 4, 5 und 6 sind als erhabene Einfassungsabschnitte 10 ausgebildet.

Fig. 2B zeigt eine Querschnittansicht entlang der in der Fig. 1 dargestellten Schnittlinie C-C' für eine andere Ausführungsform, bei der die Ausformungen einheitlich als konvexe Auswölbungen ausgebildet sind. Die Ausformungen 0 sind als konvexe Auswölbungen 13 ausgebildet, und die Einfassungsabschnitte 4, 5 und 6 sind als vertiefte Einfassungsabschnitte 12 ausgebildet.

In beiden Ausführungsformen von Fig. 2A und Fig. 2B sind die Ausformungen hinsichtlich der Wölbungsrichtung, dem Krümmungsradius der Wölbungen und der maximalen Tiefe der Wölbungen zueinander einheitlich ausgebildet.

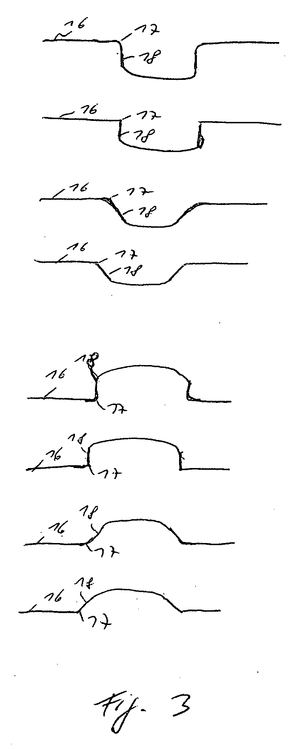

In Fig. 3 sind von oben nach unten die folgenden Ausführungsformen möglicher Ausformungen gezeigt: eine als Vertiefung ausgebildete Ausformung, die einen abgerundeten Übergangsbereich zwischen dem erhabenen Einfassungsabschnitt zu der Vertiefung aufweist, wobei die mit dem abgerundeten Übergangsbereich verbundenen Wandabschnitte einen rechten Winkel aufspannen; eine als Vertiefung ausgebildete Ausformung, die einen kantigen Übergangsbereich zwischen dem erhabenen Einfassungsabschnitt zu der Vertiefung aufweist, wobei die mit dem kantigen Übergangsbereich verbundenen Wandabschnitte einen rechten Winkel aufspannen; eine als Vertiefung ausgebildete Ausformung, die einen abgerundeten Übergangsbereich zwischen dem erhabenen Einfassungsabschnitt zu der Vertiefung aufweist, wobei die mit dem abgerundeten Übergangsbereich verbundenen Wandabschnitte einen stumpfen Winkel aufspannen; eine als Vertiefung ausgebildete Ausformung, die einen kantigen Übergangsbereich zwischen dem erhabenen Einfassungsabschnitt zu der Vertiefung aufweist, wobei die mit dem kantigen Übergangsbereich verbundenen Wandabschnitte einen stumpfen Winkel aufspannen; eine als Auswölbung ausgebildete Ausformung, die einen abgerundeten Übergangsbereich zwischen dem vertieften Einfassungsabschnitt zu der Auswölbung aufweist, wobei die mit dem abgerundeten Übergangsbereich verbundenen Wandabschnitte einen rechten Winkel aufspannen; eine als Auswölbung ausgebildete Ausformung, die einen kantigen Übergangsbereich zwischen dem vertieften Einfassungsabschnitt zu der Auswölbung aufweist, wobei die mit dem kantigen Übergangsbereich verbundenen Wandabschnitte einen rechten Winkel aufspannen; eine als Auswölbung ausgebildete Ausformung, die einen abgerundeten Übergangsbereich zwischen dem vertieften Einfassungsabschnitt zu der Auswölbung aufweist, wobei die mit dem abgerundeten Übergangsbereich verbundenen Wandabschnitte einen stumpfen Winkel aufspannen; und eine als Auswölbung ausgebildete Ausformung, die einen kantigen Übergangsbereich zwischen dem vertieften Einfassungsabschnitt zu der Auswölbung aufweist, wobei die mit dem kantigen Übergangsbereich verbundenen Wandabschnitte einen stumpfen Winkel aufspannen.

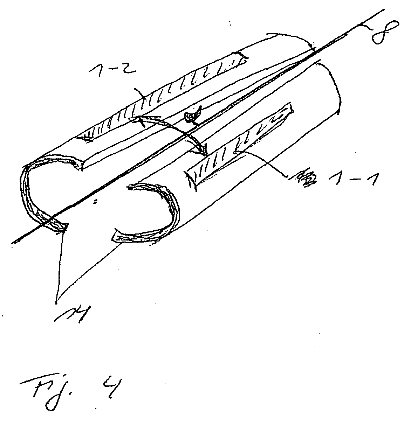

In Fig. 4 ist ein Griff eines medizinischen Instrumentes mit zwei Griffflächenteilabschnitten dargestellt. Der sich in Richtung der Längsachse des Instrumentes 8 erstreckende Griff 14 weist zwei voneinander in einem Winkel α in einer zu der Längsachse des Instrumentes 8 radialen Umfangsrichtung beabstandete Griffflächenteilabschnitte 1-1 und 1-2 auf, wobei jeder der Griffflächenteilabschnitte 1-1 und 1-2 in der radialen Umfangsrichtung auf mindestens 10 Prozent der gesamten radialen Umfangsrichtung angeordnet ist.

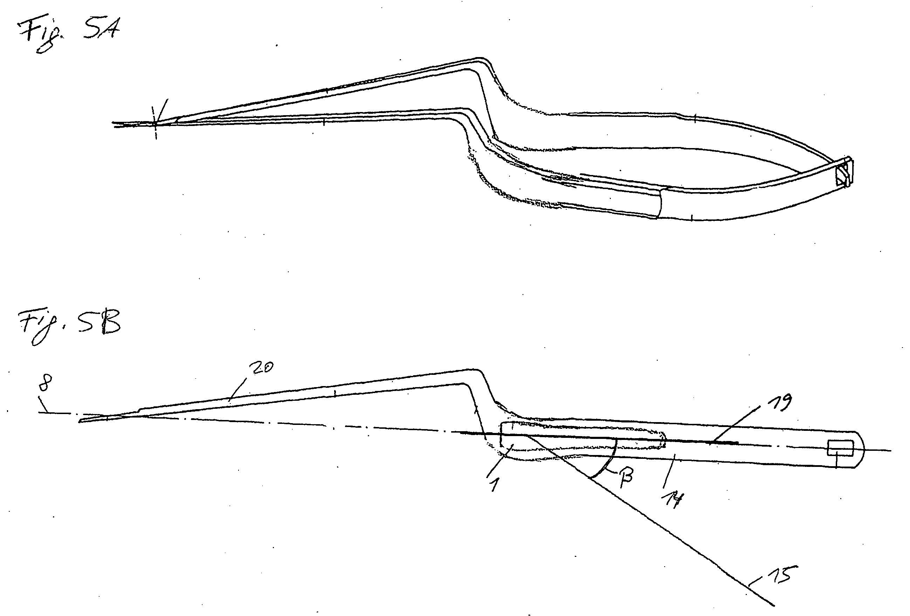

In Fig. 5A und Fig. 5B ist ein beispielhaftes erfindungsgemäßes medizinisches Instrument gezeigt. Das erfindungsgemäße medizinische Instrument weist einen Eingriffabschnitt 20 und einen Griff 19 mit einem Griffflächenabschnitt 1 auf. Eine Längsachse des Instruments 8 erstreckt sich in gleicher Richtung wie eine Längsachse des Griffs 19. Die Längsachse des Griffes 19 und eine zur Verwendung des Instrumentes vorgesehene Halteachse 15 für den Daumen spannen einen Winkel β auf.

Die beispielhaft erläuterten erfindungsgemäßen Ausführungsformen schränken den Schutzbereich der Erfindung nicht ein. Der Schutzbereich der Erfindung wird durch die angefügten Patentansprüche definiert.

### Bezugszeichenliste

- 0: Ausformung
- 1: Griffflächenabschnitt
- 1-1, 1-2: Griffflächenteilabschnitt
- 2: hexagonalförmiger Rand
- 3-1, 3-2, 3-3, 3-4, 3-5, 3-6: Randabschnitte
- 4, 5, 6: rechteckige Einfassungsabschnitte
- 7: dreieckiger Einfassungsabschnitt
- 8: Längsachse des Instrumentes
- 9: Wabenstruktur
- 10: erhabener Einfassungsabschnitt
- 11: konkave Vertiefung
- 12: vertiefter Einfassungsabschnitt
- 13: konvexe Auswölbung
- 14: Griff
- 15: zur Verwendung des Instrumentes vorgesehene Halteachse
- 16: Wandabschnitt eines Einfassungsabschnitts
- 17: Übergangsbereich
- 18: Wandabschnitt einer Ausformung
- 19: Längsachse des Griffes
- 20: Eingriffabschnitt
- α: Beabstandungswinkel der Griffflächenteilabschnitte
- β: zwischen der zur Verwendung des Instrumentes vorgesehenen Halteachse und der Längsachse des Griffes und/oder des Instrumentes aufgespannter Haltewinkel
- ϕ: von aneinander angrenzenden Randabschnitten aufgespannter Winkel

## Patentansprüche

1. Medizinisches, insbesondere chirurgisches, Instrument mit einem Eingriffabschnitt (20) für einen medizinischen Eingriff und einem zum Halten des Instrumentes, bevorzugt zur Betätigung des Eingriffabschnitts, ausgebildeten Griff (14) mit einem Griffflächenabschnitt (1) aus einem nicht rostendem Metall, der eine Anordnung mit Ausformungen (0) und zwischen den Ausformungen gebildeten Einfassungsabschnitten (4, 5, 6, 7) aufweist,
**dadurch gekennzeichnet, dass**
die Ausformungen (0) jeweils einen hexagonalförmigen Rand (2) mit sechs Randabschnitten gleicher Länge (3-1, 3-2, 3-3, 3-4, 3-5, 3-6) aufweisen, dass aneinander angrenzende der Randabschnitte (3-1, 3-2; 3-2, 3,3; ...) jeder Ausformung (0) vor und/oder nach einer Abwicklung der Anordnung auf eine ebene Fläche denselben Winkel (ϕ) von 120 Grad aufspannen, und dass der Rand (2) durch die zwischen benachbarten der Ausformungen (0) gebildeten Einfassungsabschnitte (4, 5, 6, 7) ausgebildet ist, wodurch sich eine Wabenstruktur (9) ergibt.

2. Instrument nach Anspruch 1, **dadurch gekennzeichnet, dass** die Ausformungen (0) so angeordnet sind, dass, vorzugsweise für alle der Ausformungen, ein Paar der zueinander parallelen der Randabschnitte (3-2, 3-5) einer der Ausformungen mit einer für die Verwendung des Instrumentes vorgesehenen Halteachse (15), insbesondere eine Längsachse des Griffes und/oder des Instrumentes (19, 8), einen rechten Winkel aufspannt.

3. Instrument nach einer der vorherigen Ansprüche, **dadurch gekennzeichnet, dass** die Ausformungen so angeordnet sind, dass eine Winkelhalbierende der aneinander angrenzenden der Randabschnitte (3-1, 3-2; 3-4, 3-5) der Ausformungen sich parallel zu einer Längsachse des Griffs und/oder des Instrumentes (19, 8) erstreckt.

4. Instrument nach einer der vorherigen Ansprüche, **dadurch gekennzeichnet, dass**, vorzugsweise für alle der Ausformungen, einer der Randabschnitte einer der Ausformungen zu einem benachbarten der Randabschnitte einer anderen der Ausformungen parallel verläuft, und ein zwischen diesen benachbarten parallelen Randabschnitten angeordneter Einfassungsabschnitt (4, 5, 6) der Einfassungsabschnitte eine konstante, senkrecht an den parallelen Randabschnitten gemessene Einfassungsabschnittbreite (B) aufweist.

5. Instrument nach Anspruch 4, **dadurch gekennzeichnet, dass** alle zwischen benachbarten parallelen Randabschnitten der Randabschnitte angeordneten Einfassungsabschnitte (4, 5, 6) der Einfassungsabschnitte eine konstante, vorzugsweise identische, Einfassungsabschnittbreite (B) im Bereich von 0,4 mm bis 3,7 mm, bevorzugt von 0,5 bis 3,5, noch bevorzugter von 0,6 mm bis 1,6 mm, aufweisen, und/oder jede der Ausformungen zwischen ihren jeweils gegenüberliegenden parallelen Randabschnitten (3-1, 3-4; 3-2, 3-5; 3-3, 3-6) eine konstante, vorzugsweise zu den anderen Ausformungen identische, Breite (A) im Bereich von 0,7 mm bis 5,7 mm, bevorzugt 0,8 mm bis 5,5 mm, noch bevorzugter von 1,0 mm bis 2,4 mm, aufweist.

6. Instrument nach Anspruch 5, **dadurch gekennzeichnet, dass** das Verhältnis zwischen der Breite der Ausformungen (A) und der Einfassungsabschnittbreite (B) im Bereich von 1,4 bis 1,8 liegt und bevorzugt dem Goldenen Schnitt entspricht.

7. Instrument nach einem der vorherigen Ansprüche, **dadurch gekennzeichnet, dass** die Ausformungen (0), insbesondere untereinander einheitlich, als Vertiefung (11) oder als Auswölbung (13) gegenüber einer Ebene, auf der der hexagonalförmige Rand (2) der jeweiligen der Ausformungen liegt, ausbildet sind und sich, insbesondere untereinander einheitlich, maximal im Bereich von 0,2 mm bis 1,4 mm, bevorzugt von 0,35 mm bis 1,20 mm, senkrecht zu der Ebene, auf der der hexagonalförmige Rand (2) der jeweiligen der Ausformungen liegt, erstrecken, und/oder zumindest eine Untermenge der Ausformungen (0) als Durchgangslöcher ausgebildet ist.

8. Instrument nach einem der vorherigen Ansprüche, **dadurch gekennzeichnet, dass** die Ausformungen, insbesondere untereinander einheitlich, gewölbt, insbesondere als konkave Vertiefung (11) oder als konvexe Auswölbung (13), ausbildet sind.

9. Instrument nach einem der vorherigen Ansprüche, **dadurch gekennzeichnet, dass**, insbesondere einheitlich für alle der Einfassungsabschnitte (4, 5, 6) und alle der Ausformungen (0), ein Übergangsbereich (17) von einem der Einfassungsabschnitte zu einer der Ausformungen kantig oder abgerundet ausgebildet ist, und/oder dass, insbesondere einheitlich für alle der Einfassungsabschnitte und alle der Ausformungen, ein Wandabschnitt (16) einer der Einfassungsabschnitte mit einem dazu durch den Übergangsbereich beabstandeten Wandabschnitt (18) einer der Ausformungen einen rechten oder stumpfen Winkel aufspannt.

10. Instrument nach einem der vorherigen Ansprüche, **dadurch gekennzeichnet, dass** der Griffflächenabschnitt in mindestens zwei voneinander in einem Winkel (α) von mindestens 20 Grad in einer zu einer Längsachse des Instrumentes (8) radialen Umfangsrichtung beabstandete Griffflächenteilabschnitte (1-1, 1-2) unterteilt ist, und dass jeder Griffflächenteilabschnitt in der radialen Umfangsrichtung auf mindestens 10 Prozent der gesamten radialen Umfangsrichtung angeordnet ist.

11. Instrument nach einem der vorherigen Ansprüche, **dadurch gekennzeichnet, dass** der Griffflächenabschnitt (1) in benachbarte Griffflächenteilabschnitte unterteilt ist, und einer der Griffflächenteilabschnitte ausschließlich als Vertiefungen (11) ausgebildete Ausformungen (0) aufweist, während der andere der Griffflächenteilabschnitte ausschließlich als Auswölbungen (13) ausgebildete Ausformungen (0) aufweist.

## Claims

1. A medical instrument, in particular a surgical instrument, with an intervention portion (20) for a medical intervention, and with a grip (14) designed for holding the instrument, preferably for actuating the intervention portion, with a grip surface portion (1) which is made of a stainless metal and which has an arrangement with shapings (0) and, formed between the shapings, enclosure portions (4, 5, 6, 7), **characterized in that**
the shapings (0) each have a hexagonal edge (2) with six edge portions of equal length (3-1, 3-2, 3-3, 3-4, 3-5, 3-6), that mutually adjoining edge portions (3-1, 3-2; 3-2, 3-3; ...) of each shaping (0) upstream and/or downstream of a winding of the arrangement on a plane surface span the same angle (ϕ) of 120 degrees, and that the edge (2) is formed by the enclosure portions (4, 5, 6, 7) formed between adjacent shapings (0), as a result of which a honeycomb structure (9) is obtained.

2. The instrument according to Claim 1, **characterized in that** the shapings (0) are arranged so that, preferably for all of the shapings, a pair of the edge portions (3-2, 3-5), parallel to one another, of one of the shapings spans a right angle with a holding axis (15) provided for the use of the instrument, in particular a longitudinal axis of the grip and/or of the instrument (19, 8).

3. The instrument according to one of the preceding claims, **characterized in that** the shapings are arranged so that an angle bisector of the mutually adjoining edge portions (3-1, 3-2; 3-4, 3-5) of the shapings extends parallel to a longitudinal axis of the grip and/or of the instrument (19, 8).

4. The instrument according to one of the preceding claims, **characterized in that**, preferably for all of the shapings, one of the edge portions of one of the shapings runs parallel to an adjacent one of the edge portions of another of the shapings, and an enclosure portion (4, 5, 6) of the enclosure portions, arranged between these adjacent parallel edge portions, has a constant enclosure portion width (B), measured perpendicularly on the parallel edge portions.

5. The instrument according to Claim 4, **characterized in that** all enclosure portions (4, 5, 6) of the enclosure portions arranged between adjacent parallel edge portions have a constant, preferably identical, enclosure portion width (B) in the range of 0.4 mm to 3.7 mm, preferably of 0.5 to 3.5, more preferably of 0.6 mm to 1.6 mm, and/or each of the shapings between their respectively opposite parallel edge portions (3-1, 3-4; 3-2, 3-5; 3-3, 3-6) has a constant width (A), preferably identical to the other shapings, in the range of 0.7 mm to 5.7 mm, preferably 0.8 mm to 5.5 mm, more preferably of 1.0 mm to 2.4 mm.

6. The instrument according to Claim 5, **characterized in that** the ratio between the width of the shapings (A) and the enclosure portion width (B) lies in the range of 1.4 to 1.8 and preferably corresponds to the golden ratio.

7. The instrument according to one of the preceding claims, **characterized in that** the shapings (0), in particular uniformly with respect to one another, are constructed as a depression (11) or as a bulge (13) with respect to a plane on which the hexagonal edge (2) of the respective one of the shapings lies, and, in particular uniformly with respect to one another, extend as a maximum in the range of 0.2 mm to 1.4 mm, preferably of 0.35 mm to 1.20 mm, perpendicularly to the plane on which the hexagonal edge (2) of the respective one of the shapings lies, and/or at least a subset of the shapings (0) is constructed as through-holes.

8. The instrument according to one of the preceding claims, **characterized in that** the shapings, in particular uniformly with respect to one another, are constructed so as to be curved, in particular as a concave depression (11) or as a convex bulge (13).

9. The instrument according to one of the preceding claims, **characterized in that**, in particular uniformly for all of the enclosure portions (4, 5, 6) and all of the shapings (0), a transition region (17) from one of the enclosure portions to one of the shapings is constructed in an angular or rounded manner, and/or that, in particular uniformly for all of the enclosure portions and all of the shapings, a wall portion (16) of one of the enclosure portions spans a right angle or obtuse angle with a wall portion (18) of one of the shapings spaced apart thereto by the transition region.

10. The instrument according to one of the preceding claims, **characterized in that** the grip surface portion is divided into at least two partial grip surface portions (1-1, 1-2) spaced apart from one another in an angle (α) of at least 20 degrees in a radial circumferential direction to a longitudinal axis of the instrument (8), and that each partial grip surface portion is arranged in the radial circumferential direction on at least 10 percent of the entire radial circumferential direction.

11. The instrument according to one of the preceding claims, **characterized in that** the grip surface portion (1) is divided into adjacent partial grip surface portions, and one of the partial grip surface portions has shapings (0) configured exclusively as depressions (11), whilst the other of the partial grip surface portions has shapings (0) configured exclusively as bulges (13).

## Revendications

1. Instrument médical, en particulier chirurgical, avec une partie d'intervention (20) pour une intervention médicale et une poignée (14) destinée à tenir l'instrument, de préférence pour l'actionnement de la partie d'intervention, avec une partie de face de poignée (1) en un métal inoxydable, qui présente un ensemble de déformations (0) et des parties d'encadrement (4, 5, 6, 7) formées entre les déformations, **caractérisé en ce que** les déformations (0) présentent respectivement un bord hexagonal (2) avec six parties de bord de même longueur (3-1, 3-2, 3-3, 3-4, 3-5, 3-6), **en ce que** des parties de bord adjacentes l'une à l'autre (3-1, 3-2; 3-2, 3-3; ...) de chaque déformation (0) forment, avant et/ou après développement de l'ensemble sur une surface plane, le même angle (ϕ) de 120 degrés, et **en ce que** le bord (2) est formé par des parties d'encadrement (4, 5, 6, 7) formées entre des déformations voisines (0), ce qui conduit à une structure en nid d'abeilles (9).

2. Instrument selon la revendication 1, **caractérisé en ce que** les déformations (0) sont disposées de telle manière que, de préférence pour toutes les déformations, une paire de parties de bord parallèles l'une à l'autre (3-2, 3-5) d'une des déformations forme un angle droit avec un axe de tenue (15) prévu pour l'utilisation de l'instrument, en particulier un axe longitudinal de la poignée et/ou de l'instrument (19, 8).

3. Instrument selon l'une quelconque des revendications précédentes, **caractérisé en ce que** les déformations sont disposées de telle manière qu'une bissectrice des parties de bord (3-1, 3-2; 3-4, 3-5) adjacentes l'une à l'autre des déformations s'étende parallèlement à un axe longitudinal de la poignée et/ou de l'instrument (19, 8).

4. Instrument selon l'une quelconque des revendications précédentes, **caractérisé en ce que**, de préférence pour toutes les déformations, une des parties de bord d'une des déformations s'étend parallèlement à une partie de bord voisine des parties de bord d'une autre des déformations, et une partie d'encadrement (4, 5, 6) des parties d'encadrement disposée entre ces parties de bord parallèles voisines présente une largeur de partie d'encadrement (B) constante, mesurée perpendiculairement aux parties de bord parallèles.

5. Instrument selon la revendication 4, **caractérisé en ce que** toutes les parties d'encadrement (4, 5, 6) des parties d'encadrement, disposées entre des parties de bord voisines parallèles des parties de bord, présentent une largeur de partie d'encadrement (B) constante, de préférence identique, située dans une plage de 0,4 mm à 3,7 mm, de préférence de 0,5 mm à 3, 5 mm, de préférence encore de 0,6 mm à 1,6 mm, et/ou chacune des déformations présente, entre ses parties de bord parallèles respectivement opposées (3-1, 3-4; 3-2, 3-5; 3-3, 3-6) une largeur (A) constante, de préférence identique aux autres déformations, située dans une plage de 0,7 mm à 5,7 mm, de préférence de 0,8 mm à 5,5 mm, et de préférence encore de 1,0 mm à 2, 4 mm.

6. Instrument selon la revendication 5, **caractérisé en ce que** le rapport entre la largeur des déformations (A) et la largeur de partie d'encadrement (B) se situe dans une plage de 1,4 à 1,8 et correspond de préférence au Nombre d'Or.

7. Instrument selon l'une quelconque des revendications précédentes, **caractérisé en ce que** les déformations (0) sont configurées, en particulier de façon unitaire entre elles, sous forme de dépression (11) ou de protubérance (13) par rapport à un plan sur lequel le bord hexagonal (2) de chacune de déformations est situé, et s'étendent, en particulier de façon unitaire entre elles, au maximum dans une plage de 0,2 mm à 1,4 mm, de préférence de 0,35 mm à 1,20 mm, perpendiculairement au plan sur lequel le bord hexagonal (2) de chacune des déformations est situé, et/ou au moins une minorité des déformations (0) est réalisée sous forme de trous de passage.

8. Instrument selon l'une quelconque des revendications précédentes, **caractérisé en ce que** les déformations, en particulier de façon unitaire entre elles, sont bombées, en particulier sous forme de creux concave (11) ou de protubérance convexe (13).

9. Instrument selon l'une quelconque des revendications précédentes, **caractérisé en ce que**, en particulier de façon unitaire pour toutes les parties d'encadrement (4, 5, 6) et toutes les déformations (0), une région de transition (17) d'une des parties d'encadrement à une des déformations est réalisée sous forme d'arête ou d'arrondi, et/ou **en ce que**, en particulier de façon unitaire pour toutes les parties d'encadrement et toutes les déformations, une partie de paroi (16) d'une des parties d'encadrement forme un angle droit ou un angle obtus avec une partie de paroi (18), espacée de celle-ci par la région de transition, d'une des déformations.

10. Instrument selon l'une quelconque des revendications précédentes, **caractérisé en ce que** la partie de face de poignée est divisée en au moins deux parties partielles de face de poignée (1-1, 1-2) espacées l'une de l'autre d'un angle (α) d'au moins 20 degrés dans une direction périphérique radiale par rapport à un axe longitudinal (8) de l'instrument, et **en ce que** chaque partie partielle de face de poignée est disposée, dans la direction périphérique radiale, sur au moins 10 pour cent de la direction périphérique radiale totale.

11. Instrument selon l'une quelconque des revendications précédentes, **caractérisé en ce que** la partie de face de poignée (1) est divisée en parties partielles de face de poignée voisines, et une des parties partielles de face de poignée présente exclusivement des déformations (0) en forme de creux (11), tandis que l'autre des parties partielles de face de poignée présente exclusivement des déformations (0) en forme de protubérances (13).
